# EUROPEAN PATENT APPLICATION

(11) **EP 4 249 486 A1**
(43) Date of publication of application: **27.09.2023**
(21) Application number: 22163715.0
(22) Date of filing: 23.03.2022
(51) Int. Cl.: C07D 403/12, A61K 31/4709, A61K 31/4741, C07D 403/14, C07D 405/14, A61P 31/04

(54) **ALBICIDIN DERIVATIVES, THEIR USE AND SYNTHESIS**

(71) Applicant: Technische Universität Berlin, 10623 Berlin (DE)
(72) Inventor: SÜßMUTH, Roderich, 10629 Berlin (DE); WESTON, John, 65779 Kelkheim (DE); HOMMERNICK, Kay, 10551 Berlin (DE); KULIKE, Marcel, 12277 Berlin (DE); ZBOROVSKY, Lieby, 14052 Berlin (DE); KLEEBAUER, Leonardo, 10623 Berlin (DE)
(74) Representative: Maikowski & Ninnemann Patentanwälte Partnerschaft mbB

(57) **Abstract**

The present invention relates to a chemical compound according to general formula (1)

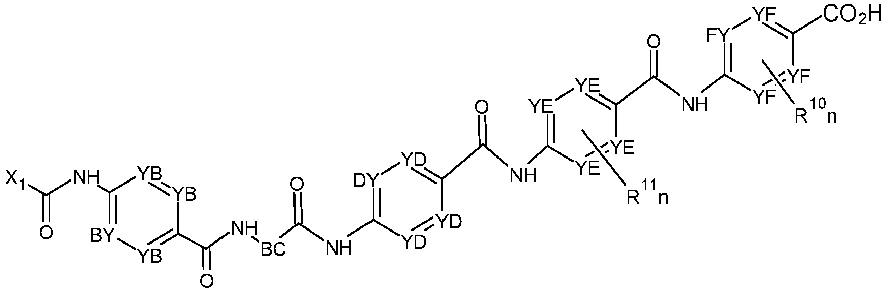

## Description

The present invention relates to albicidin derivatives.

Albicidin is a natural product, isolated from *Xanthomonas albilineans* and heterologously expressed in *Xanthomonas axonopodis* pv *vesicatoria.* Its structure (see below) is based on peptides and amino acids, but it does not contain any proteinogenic amino acids.

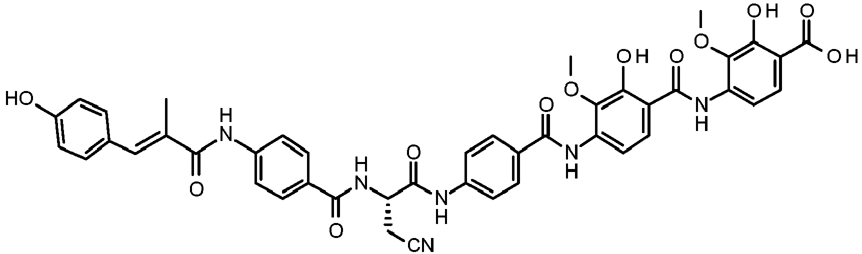

Albicidin is, on the one hand, a causative agent of the leaf scald disease in sugar cane and on the other hand a DNA-gyrase-inhibitor of prokaryotic cells (gram-positive and -negative). The mentioned properties make the natural product albicidin a potential antibiotic.

The known molecular structure of albicidin and available synthetic routes allows the development of a plurality of novel derivatives that may exhibit potential antimicrobial activities.

Since the exact interaction of albicidin with gyrase is not yet fully understood, structure-activity studies are of great importance to find out which structural elements influence the inhibition of gyrase. The aim is to develop a structure that, on the one hand, inhibits as wide a range of bacteria as possible, especially those of the ESKAPE group. On the other hand, the structure should overcome existing resistances.

Another important point is the pharmacological profile, which must be adapted so that the active substance reaches its site of action in the body. For this purpose, the active substance must ensure sufficient bioavailability, duration of action, metabolic stability, selectivity and good tolerability as well as low toxicity. These properties are determined by the so-called ADME parameters (absorption, distribution, metabolism and excretion), which in turn determine the pharmacokinetics of the substance. Decisive for optimal pharmacokinetics are physicochemical properties of the active substance such as polarity, lipophilicity and solubility.

But molecular size and flexibility also play a role, two parameters that can indirectly influence the physicochemical properties. To prevent the failure of an active ingredient at a late stage of development, it is important to integrate pharmacokinetics already in the lead structure optimization. The aim is to adjust the pharmacological profile of albicidin with regard to good oral bioavailability and optimisation of the spectrum of activity with increased bioactivity. Albicidin's poor water solubility is already evident in its structure. The calculated octanol-water partition coefficient of albicidin is clogP = 5.2.

The related property of solubility is one of the most important parameters for improving the drug. It is known that bacterial efflux systems pump out amphiphilic and lipophilic molecules more effectively than hydrophilic compounds. Moreover, in many cases, high lipophilicity is also related to increased plasma protein binding (PPB), through for example human serum albumin, lipoproteins and glycoproteins, reducing the amount of active, unbound drug. However, excessive polarity may prevent albicidin from penetrating the cytoplasmic membrane.

The problem underlying the present invention is therefore provision of new compounds, which comprise antibiotic properties, solubility and bioavailability, method of their synthesis and their use. This problem is attained by the subject-matter of the independent claims.

### Terms and Definitions

The term "purity" as used in the context of the present specification with respect to a preparation of a certain compound refers to the content of said compound relative to the sum of all compounds contained in the preparation. The term "compound" in this context is to be understood as a compound according to the invention (or any specific embodiments thereof) as well as any salts, hydrates or solvates thereof. Thus, the respective salts, hydrates or solvates are not considered as impurities according to the previous definition. The "purity" of a compound may be determined using elemental analysis, HPLC analysis using UV diode array detection also in combination with mass spectrometry detection, or quantitative NMR analysis.

The term "substituted" refers to the addition of a substituent group to a parent moiety. "Substituent groups" can be protected or unprotected and can be added to one available site or to many available sites in a parent moiety.

As used herein the term "aryl" refers to a hydrocarbon with alternating double and single bonds between the carbon atoms forming an aromatic ring structure, in particular a ten (C₁₀) membered ring. The term "heteroaryl" refers to aromatic structures comprising a ten membered ring or polyring structure, comparable to aryl compounds, in which at least one member is an oxygen or a nitrogen or a sulphur atom. Due to simplicity reasons they are denominated C₁₀ heteroaryl, wherein at least one carbon atom is replaced with an oxygen, a nitrogen or a sulphur atom forming an aromatic structure.

### Description of the invention

According to a first aspect, the invention relates to compounds having a molecular structure as defined by formula (1) 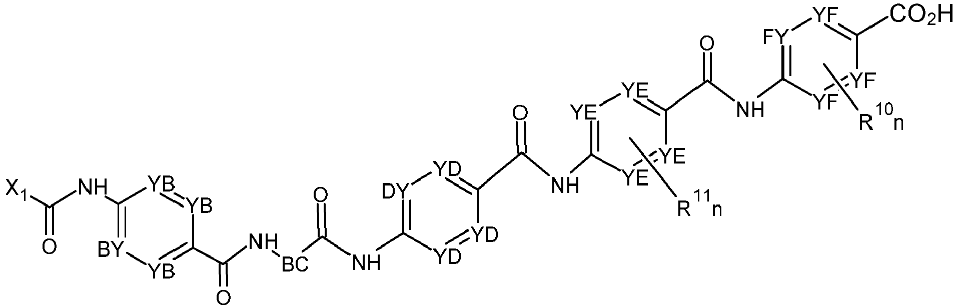
- with X₁ being
   a substituted or unsubstituted 9-10 membered bicyclic system with both rings aromatic, one ring being aromatic and the other ring containing at least one double bond or one ring being aromatic and the other ring being alicyclic,
   a substituted or unsubstituted a 9- 10 membered bicyclic heterocyclic system with both rings aromatic or one ring being aromatic and the other ring containing at least one double bond or one ring being aromatic and the other ring being alicyclic, wherein at least one heteroatom is N, S or O,

b) with BC being 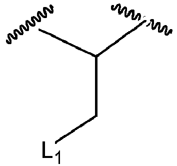 with L₁ being a substituted or unsubstituted C5-C6 aromatic heterocycle, c)
with n of R¹⁰ₙ and n of R¹¹ₙ being independently from each other 0, 1, 2, 3 or 4, in particular n of R¹⁰ₙ and n of R¹¹ₙ being 0, 1, 2 or 3, more particular n of R¹⁰ₙ and n of R¹¹ₙ being 1 and 2;
   with each R¹⁰ and R¹¹ being selected independently from any other R¹⁰ and R¹¹ from -OH, -F, -CI, -Br, -I, -CCH, -CN, -OC₁-C₆ alkyl, in particular from -OH, -F, -OCH₃, - OC₂H₅, -OiC₃H₇, -OnC₃H₇, -OCF_{3,}; and
d) with YB,YD, YE and YF being independently from each other N, CF, CCI or CH, in particular N and CH.

In one embodiment, moiety X₁ is one of the following:

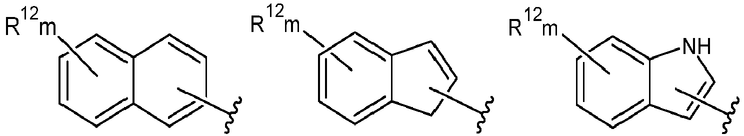

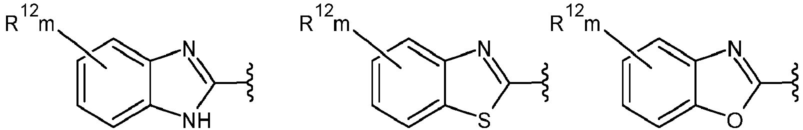

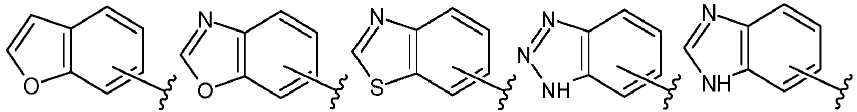

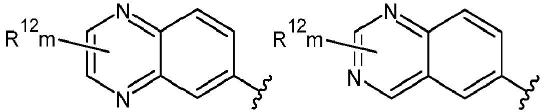

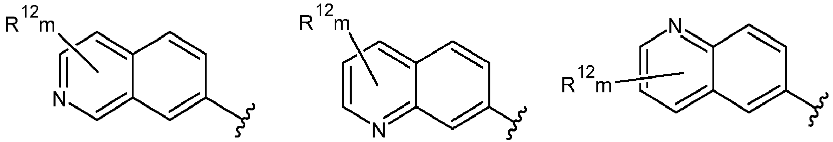

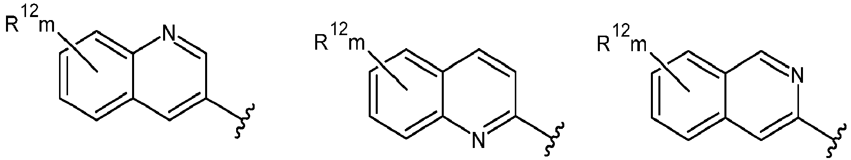

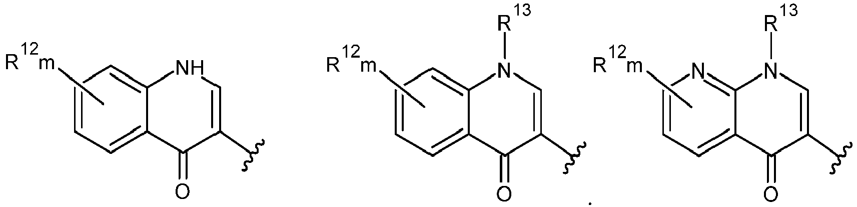

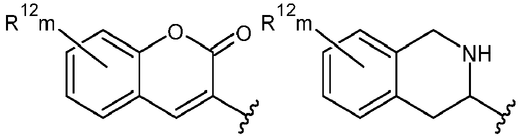

In another embodiment, moiety X₁ is one of the following

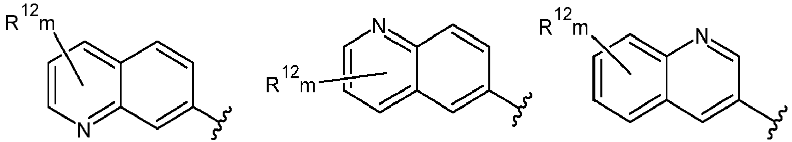

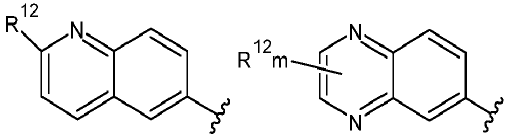

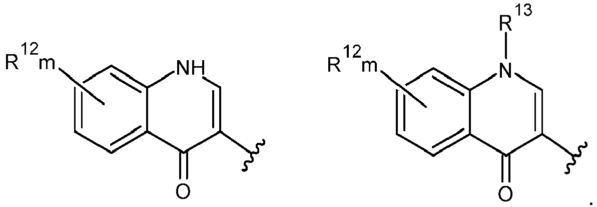

In one aspect, m of R¹² is any of 0 - 6, preferably 0, 1, 2, 3, 4, more preferably 0, 1, 2, 3; even more preferably 0, 1; and R¹² is selected from -OH, -OC₁-C₆ alkyl, -C₁-C₆ alkyl, F, -NR2,-(CH₂)ₐNR₂, -O(CH₂)ₙNR₂ with R being H or -C1-C6 alkyl and n being 1 or 2, in particular from OH, -CH₃, -C₂H₅, -OCH₃, -OC₂H₅, or two of R¹² form an acetal moiety. In an embodiment R¹² is selected from -OH, -CH₃, -C₂H₅, -OCH₃, -OC₂H₅, in particular -OH and -CH₃, or two of R¹² form -O-CH₂-O- moiety.

In a further aspect R¹³ is selected from -C₁-C₆ alkyl, in particular -CH₃, -C₂H₅, -C₃H₇.

In a preferred embodiment, moiety X₁ is one of the following

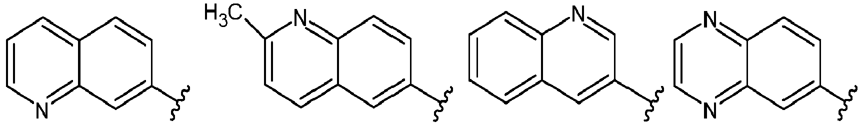

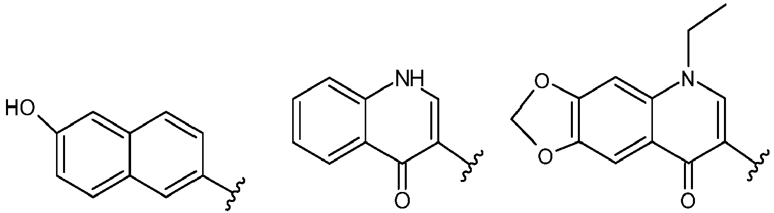

It is to be noted that in one case a naphthyl with at least one OH substituent and an indole with at least one OH-substituent may be disclaimed as moiety X1.

In one embodiment, the moiety L₁ is a five membered aromatic N-heterocycle. In a preferred embodiment, moiety L1 is an imidazole or triazole, most preferably an unsubstituted triazole. In another preferred embodiment, n of R¹⁰ₙ and n of R¹¹ₙ being 0, 1, 2, 3 or 4, in particular n of R¹⁰ₙ and n of R¹¹ₙ being 0, 1, 2 or 3, and with each R¹⁰ and with each R¹¹ independently from any other R¹⁰ being selected from -OH, -OCH₃, -OC₂H₅ or -OiPr, particularly with one R¹⁰ or R¹¹ being -OH and the other R¹⁰ or R¹¹ being -OCH₃, -OC₂H₅ or -OiPr respectively. In one specific embodiment R10 is H and R11 is one of from -OH, -OCH₃, -OC₂H₅ or -OiPr.

In a preferred embodiment the present compound may be of the general formulae (1a) 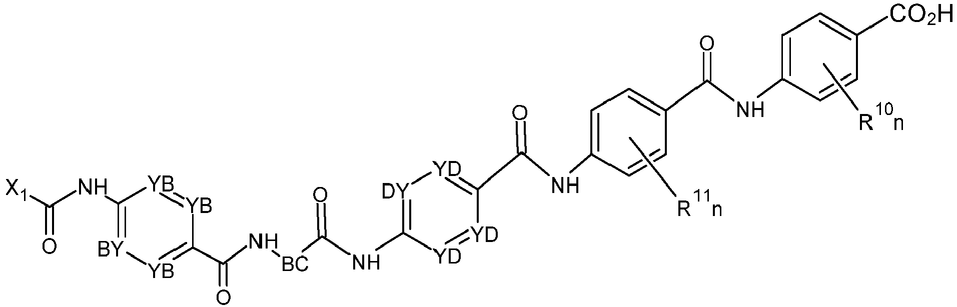
With YB being independently from each other CF, CCI or CH,
with YD being independently from each other N, CF, CCI or CH, in particular N and CH,
with X1, BC, R¹⁰n and R¹¹n as described previously above.

In a preferred embodiment the present compound may be of the general formulae (2) 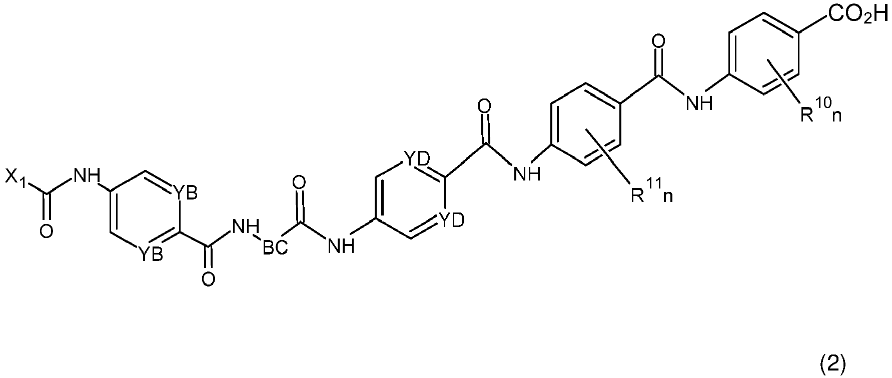
with YB being independently from each other CF, CCI or CH,
with YD being independently from each other N, CF, CCI or CH, in particular N and CH,
with X1, BC, R¹⁰n and R¹¹n as described previously above.

In a further preferred embodiment, the present compound may be of the general formulae (3) 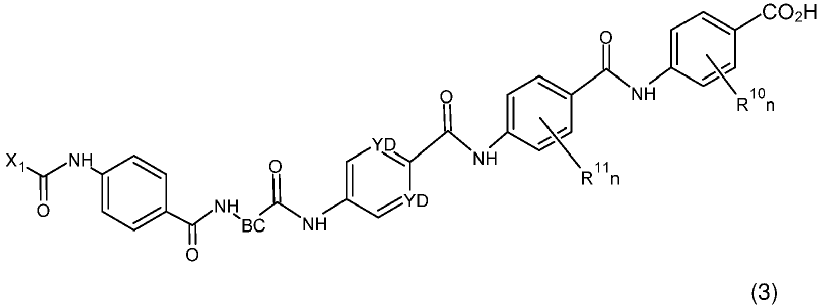 with YD being independently from each other N, CF, CCI or CH, in particular N and CH, with X1, BC, R¹⁰n and R¹¹n as described previously above.

In yet another preferred embodiment, the present compound may be of the general formulae (4) 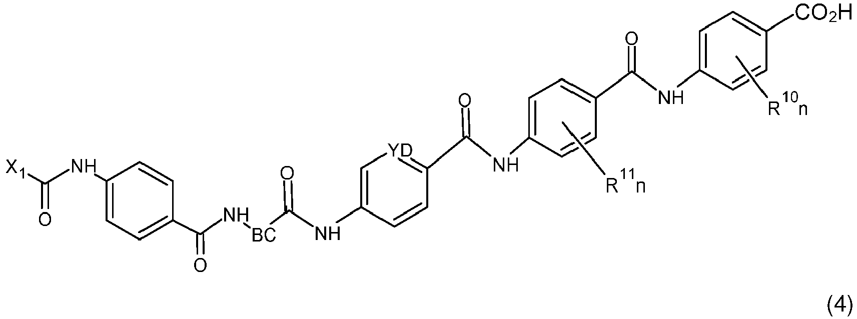
with YD being N,
with L1 being a triazole or imidazole, preferably triazole,
with X1, R¹⁰n and R¹¹n as described previously above.

In an even more preferred embodiment, the present compound may be of general formulae (5) 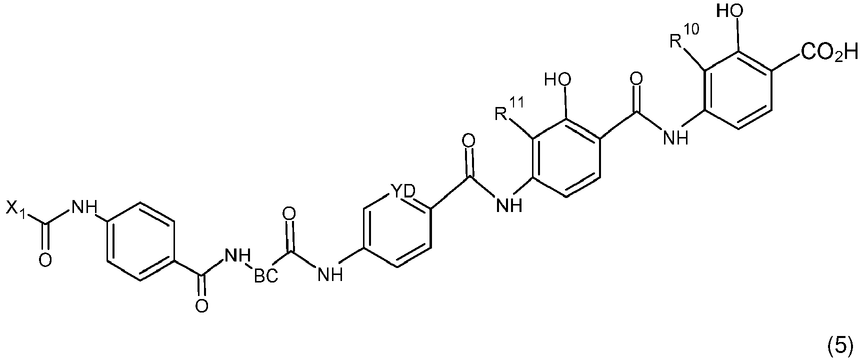
with YD being N,
with L1 being a triazole or imidazole, preferably triazole,
with X1, R¹⁰n and R¹¹n as described previously above.

In a still more preferred embodiment, the present compound may be of general formulae (6) 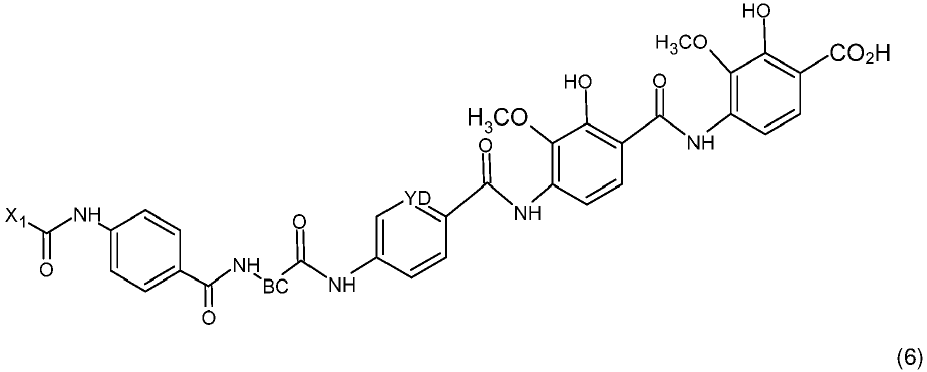
with YD being N,
with L1 being a triazole or imidazole, preferably triazole,
with X1 as described previously above.

Particular embodiments of the invention are one of the following compounds:

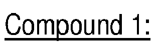

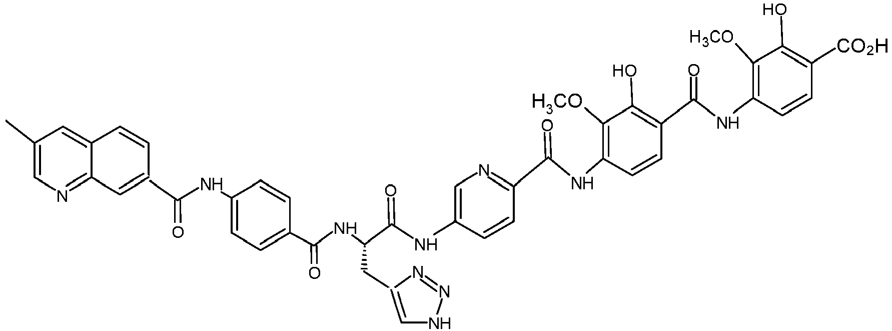

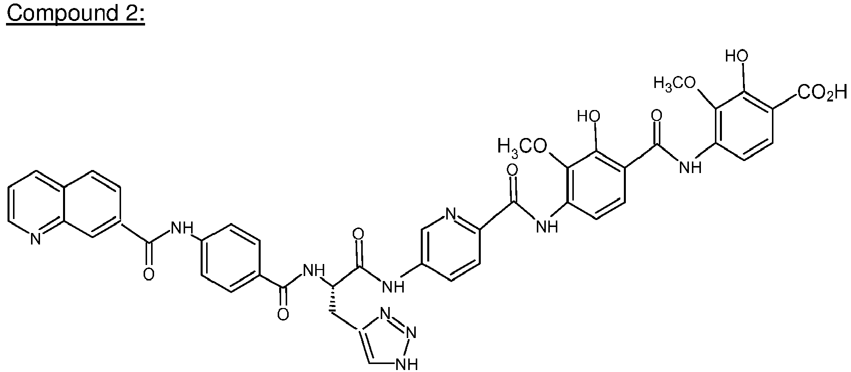

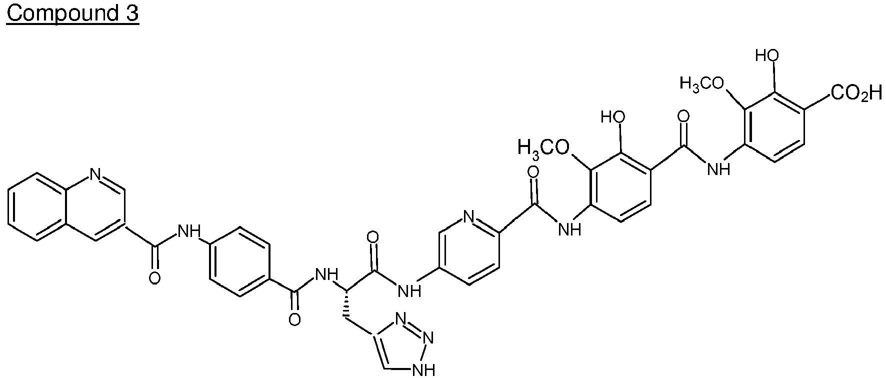

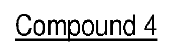

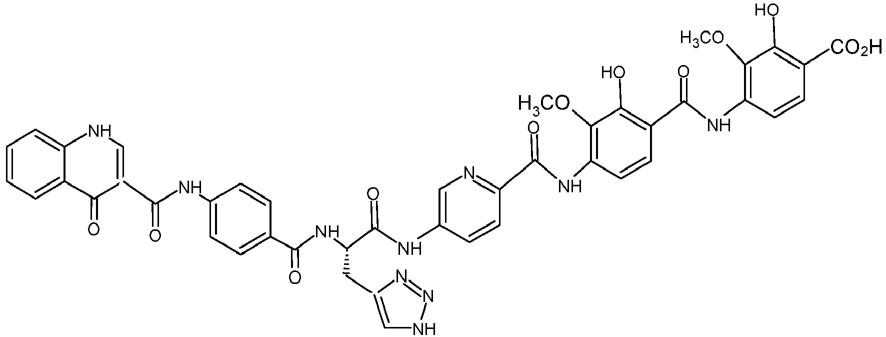

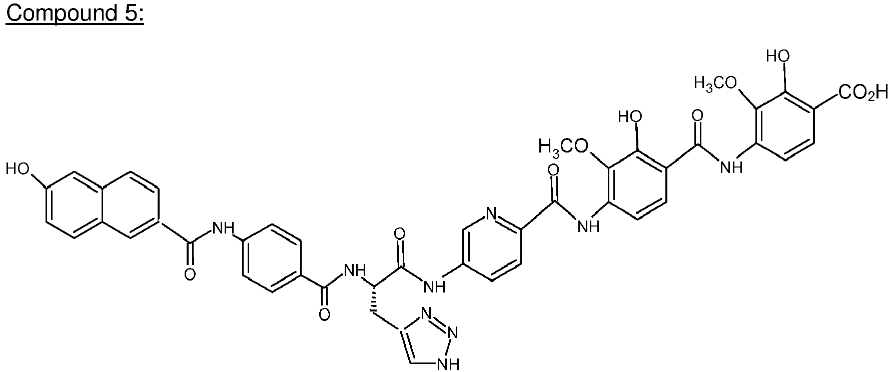

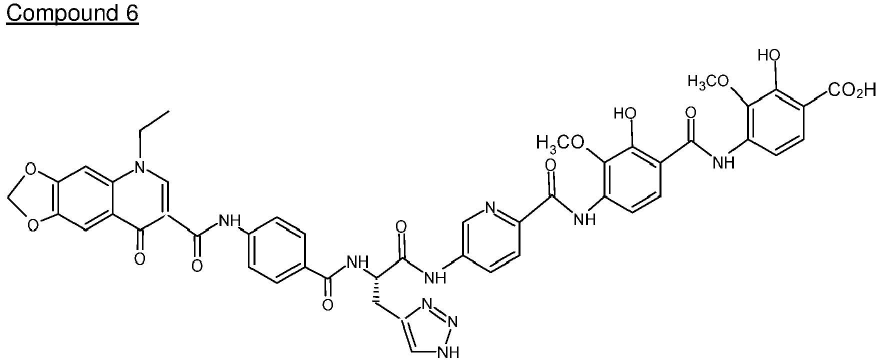

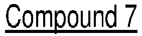

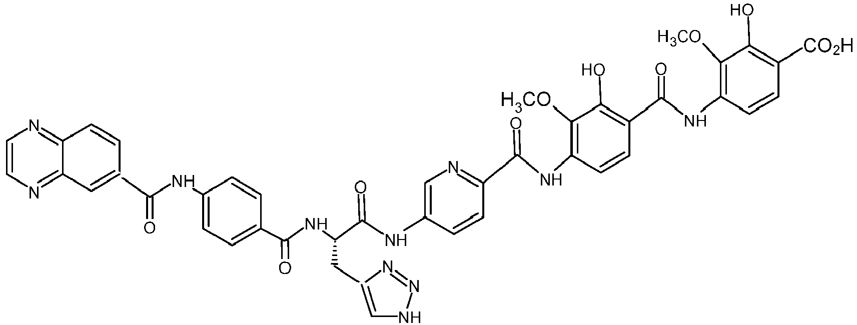

The compounds of the present invention may be used in a method of treatment of diseases, in particular for use in a method of treatment of bacterial infections caused by gram-negative or gram-positive bacterial strains.

The bacterial infection may be an infection (by a gram-negative bacterium) caused by one of the genus Acinetobacter, Bordatella, Borellia, Brucella, Camphylobacter, Chlamydia, Chlamydophila, Enterobacter, Escherichia, Francisella, Haemophilus, Helicobacter, Klebsiella, Legionella, Leptospira, Morganella Moraxella, Neisseria, Proteus, Pseudomonas, Rickettsia, Shigella, Salmonella, Stenotrophomonas, Treponema or Yersinia, in particular an infection caused by one of the genus Escherichia, Enterobacter, Salmonella, Klebsiella, Pseudomonas, Haemophilus, Shigella, Proteus or Morganella.

In a further embodiment the bacterial infection is an infection caused
- by a gram-positive bacterium, particularly an infection by one of the genus Bacillus, Clostridium , Corynebacterium, Enterococcus, Listeria, Micrococcus, Staphylococcus or Streptococcus, further in particular by one of the genus of Staphylococcus, Streptococcus, Bacillus or Micrococcus or
- by a bacterium of the family of Mycobacteriaceae, in particular of the genus Mycobacterium, further in particular an infection by one of *Mycobacterium tuberculosis, Mycobacterium leprae, Mycobacterium ulcerans* or *Mycobacterium avium,* or
- by a bacterium of the family of Mycoplasmataceae, in particular of the genus Mycoplasma, further in particular an infection by *Mycoplasma pneumonia.*

For this purpose, the present compounds may be provided in a pharmaceutical acceptable form. Pharmaceutically acceptable salts of the present compounds mean both their organic and inorganic salts as described in Remington's Pharmaceutical Sciences (17th edition, page 1418 (1985)). Because of the physical and chemical stability and the solubility, preference is given for acidic groups inter alia to sodium, potassium, calcium and ammonium salts; preference is given for basic groups inter alia to salts of maleic acid, fumaric acid, succinic acid, malic acid, tartaric acid, methylsulfonic acid, hydrochloric acid, sulfuric acid, phosphoric acid or of carboxylic acids or sulfonic acids, for example as hydrochlorides, hydrobromides, phosphates, sulfates, methanesulfonates, acetates, lactates, maleates, fumarates, malates, gluconates, and salts of amino acids, of natural bases or carboxylic acids. The preparation of pharmaceutically acceptable salts from compounds of the formula (I) which are capable of salt formation, including their stereoisomeric forms, takes place in a manner known per se. The present compounds form stable alkali metal, alkaline earth metal or optionally substituted ammonium salts with basic reagents such as hydroxides, carbonates, bicarbonates, alcoholates and ammonia or organic bases, for example trimethyl- or triethylamine, ethanolamine, diethanolamine or triethanolamine, trometamol or else basic amino acids, for example lysine, ornithine or arginine. Where the compounds of the formula (I) have basic groups, stable acid addition salts can also be prepared with strong acids. Suitable pharmaceutically acceptable acid addition salts of the compounds of the invention are salts of inorganic acids such as hydrochloric acid, hydrobromic, phosphoric, metaphosphoric, nitric and sulfuric acid, and of organic acids such as, for example, acetic acid, benzenesulfonic, benzoic, citric, ethanesulfonic, fumaric, gluconic, glycolic, isethionic, lactic, lactobionic, maleic, malic, methanesulfonic, succinic, p-toluenesulfonic and tartaric acid. The hydrochloride salt is a preferred salt.

In a preferred embodiment formulations of the present albicidin derivatives are provided which contain cyclodextrins for improving solubility of the otherwise poorly soluble albicidin derivatives. Cyclodextrins are used in a concentration of 20-40%, preferably 25-35 %, more preferably 28-30%.

Salts with a pharmaceutically unacceptable anion such as, for example, trifluoroacetate likewise belong within the framework of the invention as useful intermediates for the preparation or purification of pharmaceutically acceptable salts and/or for use in non-therapeutic, for example in vitro, applications.

The present invention furthermore relates to pharmaceutical preparations (or pharmaceutical compositions) which contain an effective amount of at least one of the present compounds and/or its pharmaceutically acceptable salts and a pharmaceutically acceptable carrier, i. e. one or more pharmaceutically acceptable carrier substances (or vehicles) and/or additives (or excipients). The pharmaceuticals can be administered orally, for example in the form of pills, tablets, lacquered tablets, coated tablets, granules, hard and soft gelatine capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carrier substances and/or additives being used in addition to the compound(s) of the formula (I) and/or its (their) pharmaceutically acceptable salts and/or its (their) prodrugs. For the production of pills, tablets, coated tablets and hard gelatine capsules it is possible to use, for example, lactose, corn starch or derivatives thereof, talc, stearic acid or its salts, etc. Carrier substances for soft gelatine capsules and suppositories are, for example, fats, waxes, semisolid and liquid polyols, natural or hardened oils, etc. Suitable carrier substances for the production of solutions, for example injection solutions, or of emulsions or syrups are, for example, water, saline, alcohols, glycerol, polyols, sucrose, invert sugar, glucose, vegetable oils, etc. Suitable carrier substances for microcapsules, implants or rods are, for example, copolymers of glycolic acid and lactic acid. The pharmaceutical preparations normally contain about 0.5 to about 90 % by weight of the present compounds and/or their pharmaceutically acceptable salts and/or their prodrugs. The amount of the active ingredient of the formula (I) and/or its pharmaceutically acceptable salts and/or its prodrugs in the pharmaceutical preparations normally is from about 0.5 to about 1000 mg, preferably from about 1 to about 500 mg.

A prodrug is a precursor chemical compound of a biological active compound of the present invention. Instead of administering the active compound or drug, a prodrug might be used instead to improve the absorption, distribution, metabolization and excretion. Prodrugs are often designed to improve bioavailability when a drug itself is poorly absorbed from the gastrointestinal tract. A prodrug may also be used to improve the selectively of the drug. This reduces adverse or unintended effects of a drug, especially important in treatments like chemotherapy, which can have severe unintended and undesirable side effects.

In addition to the active compound according to the invention and/or their pharmaceutically acceptable salts and to carrier substances, the pharmaceutical preparations can contain one or more additives such as, for example, fillers, disintegrants, binders, lubricants, wetting agents, stabilizers, emulsifiers, preservatives, sweeteners, colorants, flavourings, aromatizers, thickeners, diluents, buffer substances, solvents, solubilizers, agents for achieving a depot effect, salts for altering the osmotic pressure, coating agents or antioxidants. They can also contain two or more of the present compounds and/or their pharmaceutically acceptable salts. In case a pharmaceutical preparation contains two or more of the present compounds the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the present compounds allows a great deal of control over the biological and physico-chemical properties of the compounds and thus allows the selection of such desired compounds. Furthermore, in addition to at least one compound and/or its pharmaceutically acceptable salts, the pharmaceutical preparations can also contain one or more other therapeutically or prophylactically active ingredients. When using the present compounds the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from about 0.01 to about 100 mg/kg, preferably from about 0.1 to about 50 mg/kg, in particular from about 0.1 to about 10 mg/kg, (in each case in mg per kg of body weight). The daily dose can be divided, in particular in the case of the administration of relatively large amounts, into several, for example 2, 3 or 4, part administrations. As usual, depending on individual behaviour it may be necessary to deviate upwards or downwards from the daily dose indicated.

The compounds of the invention may also exist in various polymorphous forms, for example as amorphous and crystalline polymorphous forms. All polymorphous forms of the compounds of the invention belong within the framework of the invention and are a further aspect of the invention.

The compounds of the present invention may be present as optical isomers or as mixtures thereof. The invention relates both to the pure isomers and all possible isomeric mixtures and is hereinafter understood as doing so, even if stereochemical details are not specifically mentioned in every case. Enantiomeric mixtures of compounds of the general formula 1, which are obtainable by the process or any other way, may be separated in known manner - on the basis of the physical-chemical differences of their components - into pure enantiomers, for example by fractional crystallisation, distillation and/or chromatography, in particular by preparative HPLC using a chiral HPLC column.

According to the invention, apart from separation of corresponding isomer mixtures, generally known methods of diastereoselective or enantioselective synthesis can also be applied to obtain pure diastereoisomers or enantiomers, e.g. by carrying out the method described hereinafter and using educts with correspondingly suitable stereochemistry.

It is advantageous to isolate or synthesise the biologically more active isomer, provided that the individual compounds have different biological activities.

The present invention is explained in more detail by means of the following examples.

### Methods of synthesis

One general procedure for the synthesis of albicidin-derivatives with variations to amide bonds may comprise the steps according to the following procedure.

### Compound 1

Compound 1 is synthesized in a multistep synthesis route as follows:

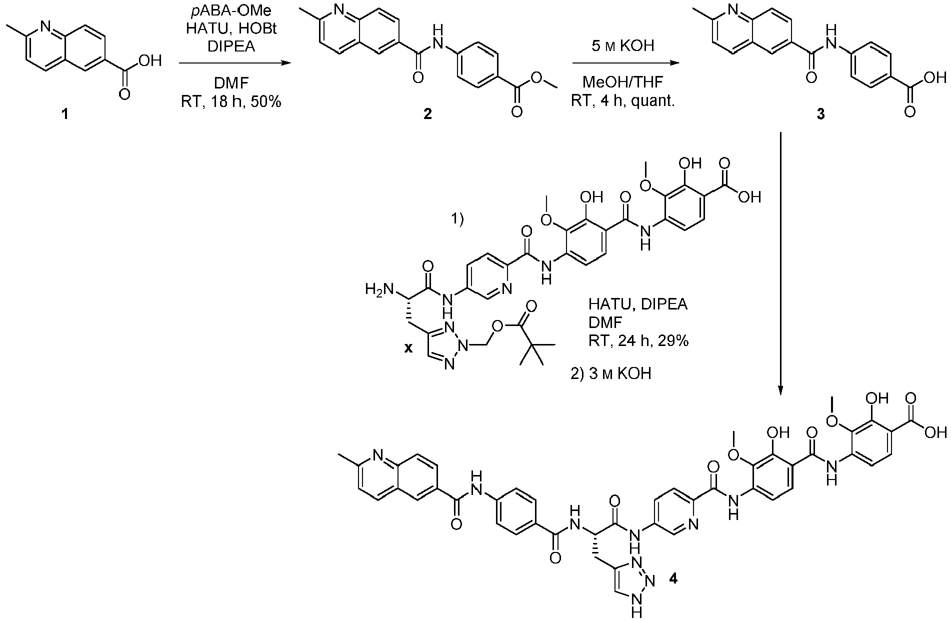

### Methyl 4-(2-methylquinoline-6-carboxamido)benzoate (2)

2-Methylquinoline-6-carboxylic acid (1, 100 mg, 534 µmol, 1.00 eq.) was dissolved in DMF (1 mL) and HOBt (36.1 mg, 267 µmol, 0.50 eq.), HATU (304 mg, 801 µmol, 1.50 eq.) and DIPEA (279 µL, 1.60 mmol, 3.00 eq.) were added and the reaction mixture was stirred for 1 h at room temperature. 4-Methyl-aminobenzoate (121 mg, 801 µmol, 1.50 eq.) was added and the reaction mixture was stirred 18 h at room temperature. The reaction mixture was diluted by EtOAc (30 mL) and the organic layer was washed by a saturated aqueous NaHCO₃ solution (3 × 20 mL) and by a saturated aqueous NaCl solution (1 × 20 mL). The organic layer was dried over MgSO₄, filtered and the solvent was removed under reduced pressure by rotary evaporation. The crude material was purified by flash column chromatography (SiO₂, EtOAc/Hex 1:1) and afforded methyl 4-(2-methylquinoline-6-carboxamido)benzoate (2, 85.0 mg, 267 µmol, 50%) as a white solid. ¹H NMR (400 MHz, DMSO-de) □ ppm 2.71 (s, 3 H) 3.85 (s, 3 H) 7.54 (d, J=8.53 Hz, 1 H) 7.99 (s, 4 H) 8.04 (d, J=8.78 Hz, 1 H) 8.22 (dd, J=8.78, 2.01 Hz, 1 H) 8.42 (d, J=8.53 Hz, 1 H) 8.60 (d, J=1.76 Hz, 1 H) 10.77 (s, 1 H) ¹³C NMR (DMSO-d₆ ,101MHz): □ = 165.8, 165.6, 161.0, 148.5, 143.6, 137.1, 131.5, 130.1, 128.4, 128.0, 125.3, 124.4, 123.1, 119.6, 51.9, 38.2, 25.0 ppm. HRMS (ESI): m/z calculated for C₁₉H₁₆N₂O₃ (M+H)⁺: 321.1229, found 321.1234.

### 4-(2-Methylquinoline-6-carboxamido)benzoic acid (3)

Methyl 4-(2-methylquinoline-6-carboxamido)benzoate (85.0 mg, 267 µmol, 1.00 eq.) was dissolved in MeOH/THF (2 mL, 1:1) and 5 M KOH solution (1 mL) was added and the reaction mixture was stirred for 18 h at room temperature. The volatiles were removed under reduced pressure by rotary evaporation and a 3 M HCI solution (2 mL) was added. The precipitated solid was filtered and washed by 1 M HCI solution. After drying at high vacuum 4-(2-methylquinoline-6-carboxamido)benzoic acid (3, 81.0 mg, 267 µmol, 100%) was obtained as brownish solid. HRMS (ESI): m/z calculated for C₁₈H₁₄N₂O₃ (M+H)⁺: 307.1074, found 307.1077.

### Compound 1 (AF-ACD-018 (4))

HATU (67.0 mg, 176 µmol, 1.35 eq.) was added to a solution of AB building block 3 (51.9 mg, 169 µmol, 1.30 eq.) in anhydrous DMF (1 mL) and the resulting solution was stirred at r.t for 45 min. A solution of tetrapeptide X (94.0 mg, 130 µmol, 1.00 eq.) and DIPEA (136 µL, 780 µmol, 6.00 eq.) in anhydrous DMF (1 mL) was added dropwise and the reaction mixture was stirred at r.t for 16 h. All volatiles were removed in vacuo and the residue was taken up in a mixture of THF (1 mL) and MeOH (1 mL), and 3 N KOH(aq.) (1 mL) was added dropwise. After 45 min of stirring, 3 N HCl(aq.) (1.1 mL) was added and the resulting suspension was evaporated under reduced pressure. The crude material was dissolved in DMSO, centrifuged, and the supernatant purified by HPLC (PLRP-S column, CH3CN in H2O). The title compound 4 (13 mg, 9% over two steps) was obtained as a colourless solid. ¹H NMR (DMSO-d₆ ,700MHz): δ (ppm) = 11.71 (br. s., 1 H), 11.58 (br. s., 1 H), 11.12 (s, 1 H), 10.86 (s, 1 H), 10.82 (s, 1 H), 10.49 (s, 1 H), 8.94 - 9.01 (m, 1 H), 8.84 (d, *J*=7.5 Hz, 2 H), 8.77 (s, 1 H), 8.40 (d, *J*=8.8 Hz, 1 H), 8.34 (dd, *J*=8.5, 2.1 Hz, 1 H), 8.21 (d, *J*=8.5 Hz, 1 H), 8.17 (d, *J*=8.8 Hz, 1 H), 8.11 (d, *J*=8.8 Hz, 1 H), 8.03 (d, *J*=9.0 Hz, 1 H), 7.92 - 7.98 (m, 4 H), 7.88 (d, *J*=9.0 Hz, 1 H), 7.82 (d, *J*=8.1 Hz, 1 H), 7.72 (br. s., 1 H), 7.59 (d, *J*=8.8 Hz, 1 H), 4.96 (br. s., 2 H), 3.92 (s, 3 H), 3.88 (s, 3 H), 3.34 (dd, *J*=14.8, 5.7 Hz, 1 H), 3.28 (dd, *J*=14.8, 9.3 Hz, 1 H), 2.86 ppm (s, 3 H) H,C-HSQC NMR (DMSO-d₆ ,101 MHz): δ (ppm) =172.4, 171.6, 171.6, 171.6, 171.5, 166.4, 166.4, 165.3, 165.3, 165.2, 163.9, 163.8, 161.8, 160.9, 154.8, 150.0, 143.9, 143.8, 142.3, 140.0, 139.9, 139.9, 139.9, 139.3, 139.2, 138.2, 137.8, 137.7, 137.7, 136.6, 136.6, 136.6, 135.9, 133.6, 130.8, 129.1, 129.1, 129.0, 129.0, 127.6, 127.6, 127.6, 126.1, 126.1, 126.1, 124.3, 119.8, 119.8, 119.8, 119.8, 115.9, 115.9, 110.8, 110.8, 110.8, 110.7, 110.7, 110.7, 109.4, 60.6, 60.6, 54.7, 54.6, 39.9, 27.5, 23.4, 23.4 ppm. HRMS (ESI): m/z calculated for C₄₅H₃₈N₁₀O₁₁ (M+H)+: 895.2786, found 895.2794.

The following compounds are obtained in an analogous synthesis procedure.

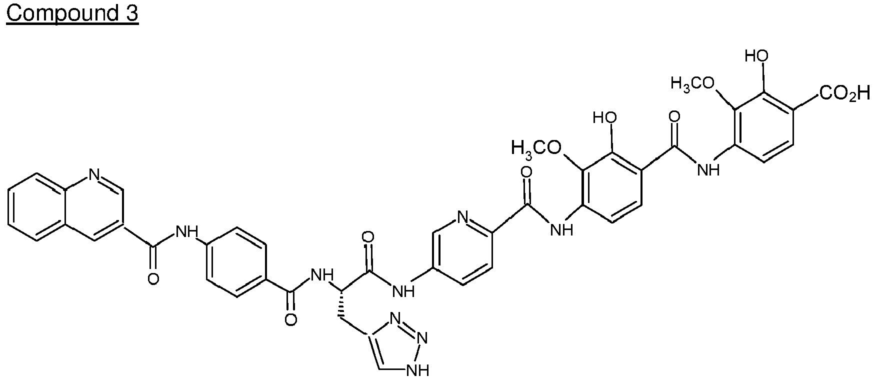

¹H NMR (700 MHz, DMSO-d6 from HSQC-ed) δ (ppm) 11.73 (s, 1H), 11.60 (s, 1H), 11.14 (s, 1H), 10.86 (d, J = 7.2 Hz, 2H), 10.50 (s, 1H), 9.39 (d, J = 2.2 Hz, 1H), 9.00 (dd, J = 17.0, 2.3 Hz, 2H), 8.85 (d, J = 7.4 Hz, 1H), 8.35 (dd, J = 8.6, 2.4 Hz, 1H), 8.22 (d, J = 8.6 Hz, 1H), 8.18 (dd, J = 8.1, 1.4 Hz, 1H), 8.17 - 8.10 (m, 2H), 8.04 (d, J = 8.9 Hz, 1H), 7.98 - 7.90 (m, 5H), 7.89 (d, J = 8.9 Hz, 1H), 7.75 (ddd, J = 8.1, 6.8, 1.2 Hz, 1H), 7.72 (s, 1H), 7.66 - 7.50 (m, 3H), 4.96 (q, J = 7.5 Hz, 1H), 3.93 (s, 3H), 3.88 (s, 3H), 3.35 (dd, J = 14.7, 5.7 Hz, 1H), 3.28 (dd, J = 14.8, 9.2 Hz, 1H).

¹³C NMR (176 MHz, DMSO from HSQC-ed) δ (ppm) 149.51, 149.51, 139.99, 139.96, 136.76, 132.05, 132.03, 129.73, 129.26, 129.23, 128.92, 128.10, 127.74, 126.99, 126.14, 123.46, 119.87, 110.88, 110.77, 61.32, 60.68, 54.80.

HRMS (ESI): m/z calcd for C₄₄H₃₇N₁₀O₁₁ [M+H]⁺ 881.2638; found 881.2642, tR = 8.32 min.

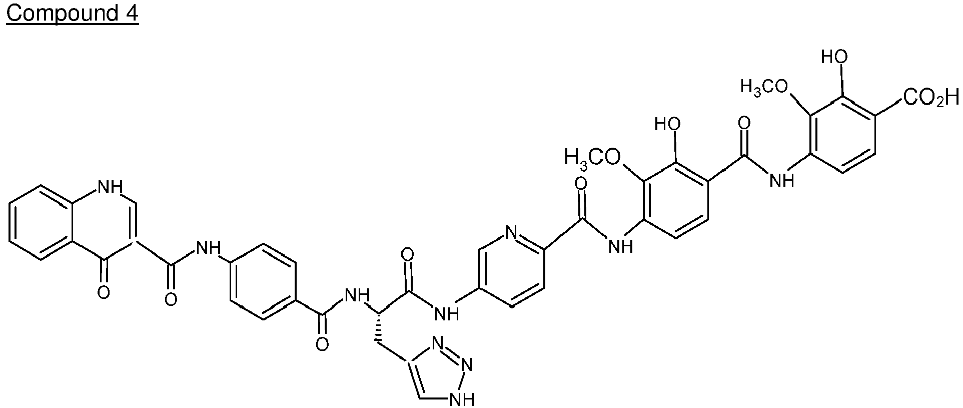

¹H NMR (DMSO-d₆, 700MHz): δ (ppm) = 13.09 (br. s., 1 H), 12.73 (s, 1 H), 11.76 (br. s., 1 H), 11.11 (s, 1 H), 10.85 (s, 1 H), 10.82 (s, 1 H), 10.49 (s, 1 H), 8.97 (s, 1 H), 8.90 (d, *J*=6.1 Hz, 1 H), 8.83 (br. s., 1 H), 8.34 (d, *J*=8.4 Hz, 1 H), 8.20 (d, *J*=8.4, 1 H), 8.10 (d, *J*=8.8 Hz, 1 H), 8.00 (d, *J*=8.4 Hz, 1 H), 7.92 (d, *J*=8.4 Hz, 2 H), 7.88 - 7.82 (m, 4 H), 7.77 (br. s., *J*=8.2 Hz, 1 H), 7.66 (br. s., 1 H), 7.58 - 7.53 (m, 2 H), 4.94 (dd, *J*¹=6.8 Hz, *J*²=13.5 Hz, 1 H), 3.91 (s, 3 H), 3.87 (s, 3 H), 3.29 - 3.24 ppm (m, 2 H).

H,C-HSQC NMR (DMSO-d₆ ,101MHz): δ (ppm) =144.6, 140.0, 133.6, 133.4, 129.3, 127.7, 127.0, 126.0, 125.9, 123.5, 119.7, 119.2, 119.1, 110.7, 110.6, 61.3, 60.8, 54.8, 27.8 ppm.

HRMS (ESI): m/z calculated for C₄₅H₃₈N₁₀O₁₁ (M+H)+: 897.2509, found 897.2563.

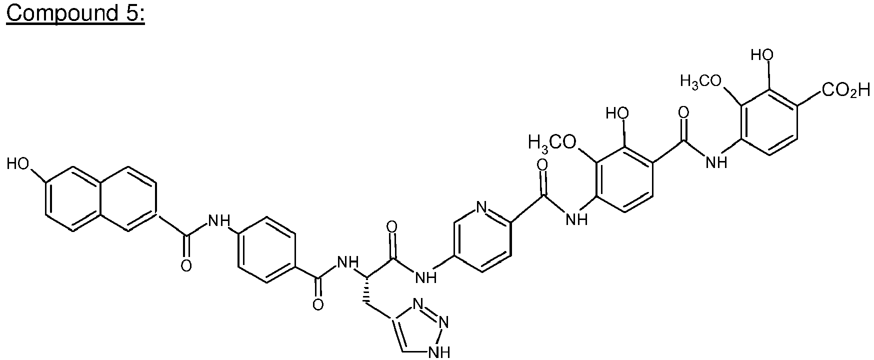

¹H NMR (700 MHz, DMSO-d₆) δ 14.66 (s, 3H), 11.72 (s, 3H), 11.13 (s, 3H), 10.85 (s, 5H), 10.51 (d, J = 16.9 Hz, 8H), 10.11 (s, 4H), 8.81 (s, 4H), 8.72 (s, 1H), 8.50 (d, J = 1.8 Hz, 4H), 8.35 (dd, J = 8.5, 2.4 Hz, 4H), 8.22 (d, J = 8.6 Hz, 4H), 8.12 (d, J = 8.9 Hz, 4H), 8.03 (d, J = 8.8 Hz, 3H), 7.95 (d, J = 3.7 Hz, 4H), 7.96 - 7.90 (m, 21H), 7.89 (d, J = 8.8 Hz, 3H), 7.82 (d, J = 8.7 Hz, 4H), 7.77 (s, 0H), 7.68 (s, 4H), 7.60 (d, *J* = 8.8 Hz, 3H), 7.23 - 7.18 (m, 8H), 7.16 (s, 1H), 7.09 (s, 1H), 7.02 (s, 1H), 4.95 (d, *J* = 7.6 Hz, 5H), 3.90 (d, *J* = 6.5 Hz, 1H), 3.88 (s, 11H). ¹³C NMR (176 MHz, DMSO from HSQC-ed) δ 139.95, 131.32, 128.83, 128.70, 127.63, 126.99, 126.61, 126.16, 125.14, 123.45, 120.05, 119.73, 110.74, 109.15, 63.80, 61.26, 60.69.

HRMS (ESI): m/z calcd for C₄₅H₃₈N₉O₁₂ [M+H]⁺ 896.2634; found 896.2643, tR = 8.38 min

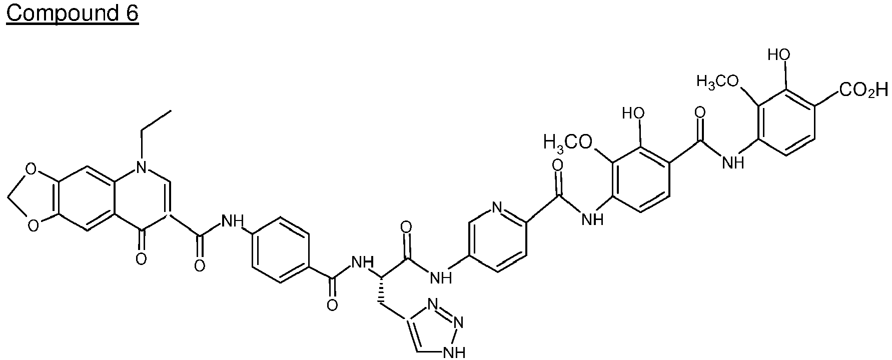

¹H NMR (DMSO-d₆ ,700MHz): δ (ppm) = 12.88 (s, 1 H), 11.72 (br. s., 1 H), 11.63 (br. s., 1 H), 11.58 (br. s., 1 H), 11.13 (s, 1 H), 10.84 (s, 1 H), 10.50 (s, 1 H), 8.98 (m, 1 H), 8.90 (s, 1 H), 8.83 (br. s., 1 H), 8.34 (d, *J*=8.7, 1 H), 8.21 (d, *J*=8.6 Hz, 1 H), 8.12 (d, *J*=8.9 Hz, 1 H), 8.03 (d, *J*=8.9 Hz, 1 H), 7.93 (d, *J*=8.6 Hz, 2 H), 7.89 (d, *J*=8.9 Hz, 1 H), 7.83 (d, *J*=8.7 Hz, 2 H), 7.72 (s, 1 H), 7.67 (br. s., 1 H), 7.60 - 7.59 (m, 2 H), 6.28 (s, 1 H), 4.94 (dd, *J*¹=7.0 Hz, *J*²=14.6, Hz, 1 H), 4.53 (q, *J*=7.1 Hz, 2 H), 3.92 (s, 3 H), 3.88 (s, 3 H), 3.34 (dd, *J*¹=14.8, *J*²=5.7 Hz, 1 H), 3.29 - 3.24 (m, 2 H), 1.40 ppm (t, *J*=7.1 Hz, 3 H).

H,C-HSQC NMR (DMSO-d₆ ,101MHz): δ (ppm) = 147.1, 140.0, 129.3, 127.6, 127.1, 126.1, 123.6, 119.2, 110.8, 110.6, 103.4, 103.0, 97.4, 61.4, 60.7, 54.7, 49.4, 27.8, 15.1 ppm. HRMS (ESI): m/z calculated for C₄₅H₃₈N₁₀O₁₁ (M+H)+: 969.2786, found 969.2798.

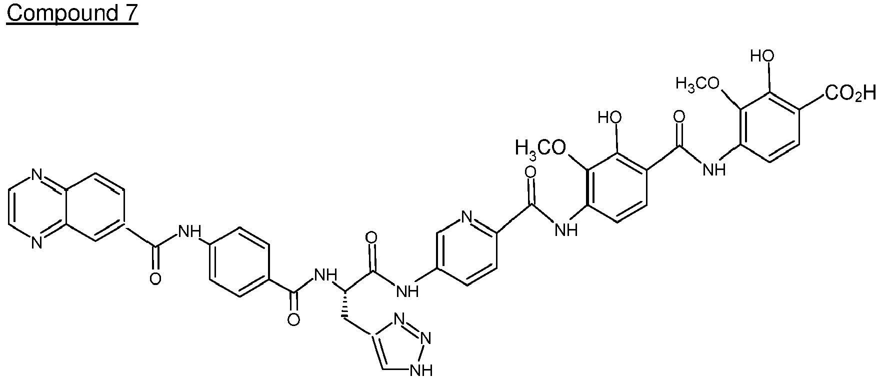

¹H NMR (DMSO-d₆ ,700MHz): δ (ppm) = 11.71 (s, 1 H), 11.58 (br. s., 1 H), 11.14 (s, 1 H), 10.87 (s, 1 H), 10.86 (s, 1 H), 10.50 (s, 1 H), 9.10 (d, *J*=1.7 Hz, 1 H), 9.08 (d, *J*=1.7 Hz, 1 H), 8.99 (s,1 H), 8.85 (d, *J*=7.5 Hz, 1 H), 8.80 (s, 1 H), 8.38 (d, *J*=8.7 Hz, 1 H), 8.35 (d, *J*=8.6, 1 H), 8.26 (d, *J*=8.8 Hz, 1 H), 8.22 (d, *J*=8.8 Hz, 1 H), 8.12 (d, *J*=8.9 Hz, 1 H), 8.04 (d, *J*=8.8 Hz, 1 H), 7.98 - 7.95 (m, 4 H), 7.89 (d, *J*=8.7 Hz, 1 H), 7.73 (br. s., 1 H), 7.60 (d, *J*=8.8 Hz, 1 H), 4.96 (dd, *J*¹=7.5 Hz, *J*²=14.7 Hz, 1 H), 3.93 (s, 3 H), 3.89 (s, 3 H), 3.35 (dd, *J*¹=5.9 Hz, *J*²=14.9 Hz, 1 H), 3.28 (dd, *J*¹=9.0 Hz, *J*²=14.6 Hz, 1 H)

H,C-HSQC NMR (DMSO-d₆ ,101MHz): δ (ppm) = 147.5, 140.0, 130.0, 129.5, 129.4, 128.9, 127.7, 126.1, 123.4, 120.0, 110.9, 110.7, 61.3, 60.8, 54.8, 27.7.

HRMS (ESI): m/z calculated for C₄₅H₃₈N₁₀O₁₁ (M+H)+: 882.2590, found 882.2588.

### Test for biological activity

### Strains:

*E. coli* DSM 1116; *E*. *coli* BW25113, S. *typhimurium* TA100; *Bacillus subtilis* DSM10; *Micrococcus luteus* DSM1790 and *M. phlei* DSM 750

### Biological testing:

The tests were performed using the micro dilution method.

### Microdilution assay:

The determination of MIC values was performed according to the ninth edition of the Approved Standard M07-A9 (CLSI. Methods for Dilution Antimicrobial Susceptibility Tests for Bacteria That Grow Aerobically; Approved Standard-Ninth Edition. CLSI document M07-A9. Wayne, PA: Clinical and Laboratory Standards Institute; 2012.)

The test was carried out for different bacterial strains (E.coli DSM 1116, *E. coli* BW25113 [gram negative], B. subtilis DSM 10 [gram positive], M. luteus DSM 1790 [gram positive], S. typhimurium TA100 [gram negative]) and *M. phlei* DSM 750 . 20 µL of cryo stock of each strain were inoculated in 20 mL of LB media (Lysogeny broth: 10 g/L peptone, 5 g/L yeast extract, 5 g/L NaCl) followed by incubation over night at 37°C, 200 rpm. The test inoculum was adjusted by the 0.5 McFarland Standard (OD625 from 0.08 to 0.1). Within 15 min of preparation, the adjusted inoculum suspension was diluted in MHBII media (BBL TM Mueller-Hinton Broth II, Becton, Dickinson and Company, New Jersey/USA) so that each well contained approximately 5 × 105 CFU/mL in a final volume of 100 µL. 95 µL of the inoculum were applied per well and 5 µL of the (diluted) antibiotic substance were added.

Previously the dry antibiotic compounds were dissolved in DMSO (100%) with a concentration of 2560 µg/mL and the resulting stock solutions were further diluted in DMSO (100%). 5 µL of each antibiotic dilution were applied to the microdilution tray to reach final concentrations of 64 µg/mL to 0.008 µg/mL. One row of each well plate was left as a growth control without antibiotic substances and another row of the microdilution tray was used as sterility control (only MHB II-media). The antimicrobial effect of the solvent (DMSO) was tested by adding 5 µL DMSO to several wells without antibiotics. Purity check and cell titer control were performed according to International Standard M07-A9 on Mueller-Hinton II Agar (Mueller Hinton II Broth, 15 g/L agar-agar). Both microdilution trays and agar plates were incubated at 37°C for 20 h and subsequently analyzed visually.

### Retention time

The product was dissolved in DMSO, centrifuged, and the supernatant injected into an HPLC (PLRP-S column, CH₃CN in H₂O). RT (Retention Time) is measured in minutes.

### Clog P

The clogP was calculated using the Chemdraw software

The results are summarized in table 1. The potency of an antibiotic is determined by the minimum inhibitory concentration (MIC). Contrary to intuition, a particularly low value is equated with a high potency.

AzaHis is used as reference compound and is of the following structure:

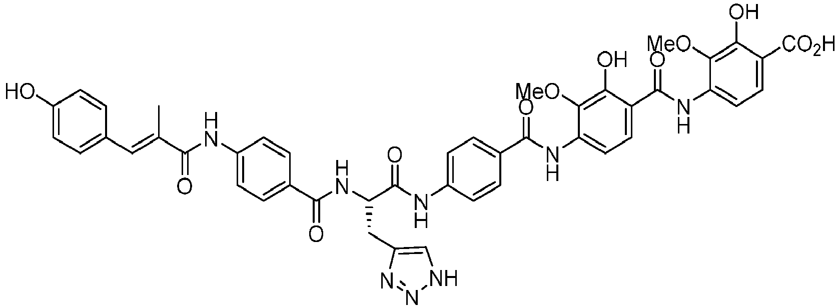

**Table 1: Antibacterial activity of compounds according to the invention against selected strains**

| **Compound** | **AzaHis** | **5** | **3** | **7** | **4** | **6** | **1** |
|---|---|---|---|---|---|---|---|
| **Strain** | | | | | | | |
| **E.Coli BW25113** | **0.016** | **0.016** | **0.016** | 0.031 | 0.063 | **0.016** | **0.016** |
| **E.Coli DSM1116** | **0.016** | **0.016** | 0.031 | 0.031 | 0.031 | **0.016** | **0.016** |
| **S.Typhimirium TA100** | **0.016** | **0.016** | 0.016 | 0.016 | 0.016 | 0.016 | 0.016 |
| **B.Subtilis DSM10** | **0.031** | 0.063 | 0.125 | 0.125 | **0.016** | **0.031** | 0.063 |
| **M.Luteus DSM1790** | **0.031** | **0.031** | 0.125 | 0.25 | **0.016** | **0.016** | 0.063 |
| **M.Phlei DSM 750** | **0.125** | 1 | 1 | 2 | 8 | **0.25** | **0.5** |
| **50% human serum** | **0.5** | 2 | 2 | 1 | 2 | **0.5** | **0.5** |
| **clogP** | 4.23 | 4.31 | 3.79 | 3.82 | 3.61 | 3.69 | 4.26 |
| **RT (min)** | 8.15 | 8,15 | 8.07 | 7.71 | 7.98 | 8.45 | **6.94** |

## Claims

1. Compound **characterized by** the general formula (1) 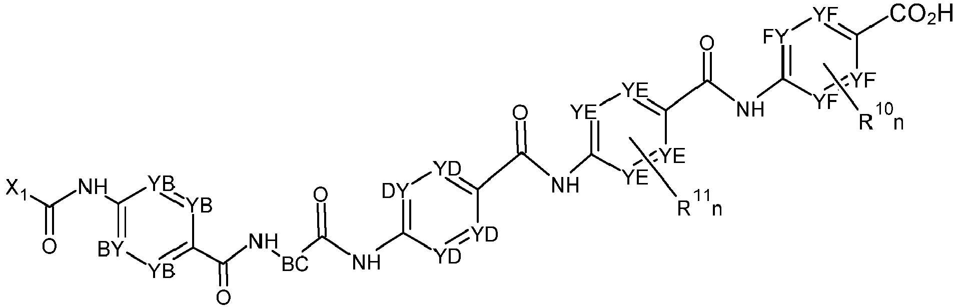
- with X₁ being
- a substituted or unsubstituted 9-10 membered bicyclic system with both rings aromatic or one ring being aromatic and the other ring containing at least one double bond or one ring being aromatic and the other ring being alicyclic,
- a substituted or unsubstituted a 9- 10 membered bicyclic heterocyclic system with both rings aromatic or one ring being aromatic and the other ring containing at least one double bond or one ring being aromatic and the other ring being alicyclic, wherein at least one heteroatom is N, S or O,
b) with BC being 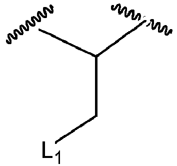 with L₁ being a substituted or unsubstituted C5-C6 aromatic heterocycle,
c) with n of R¹⁰ₙ and n of R¹¹ₙ being independently from each other 0, 1, 2, 3 or 4, in particular n of R¹⁰ₙ and n of R¹¹ₙ being 0, 1, 2 or 3, more particular n of R¹⁰ₙ and n of R¹¹ₙ being 1 and 2;
with each R¹⁰ and R¹¹ being selected independently from any other R¹⁰ and R¹¹ from -OH, -F, -CI, -Br, -I, -CCH, -CN, -OC₁-C₆ alkyl, in particular from -OH, -F, -OCH₃, - OC₂H₅, -OiC₃H₇, -OnC₃H₇, -OCF_{3,}; and
d) with YB, YD , YE and YF being independently from each other N, CF, CCI or CH, in particular N and CH.

2. Compound according to claim 1, **characterized in that** X₁ is one of the following: 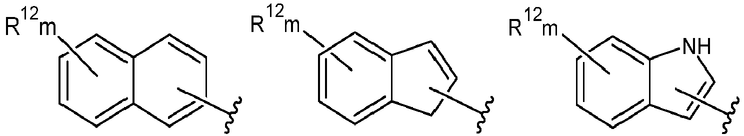 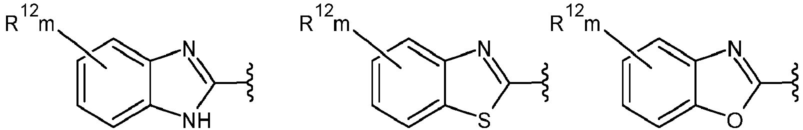 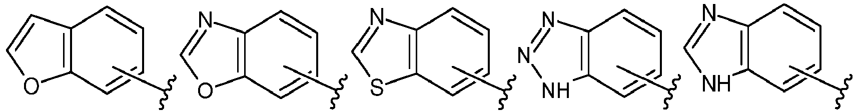 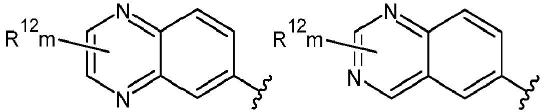 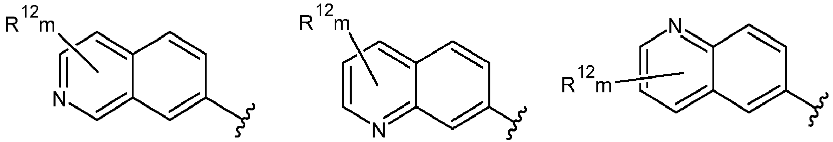 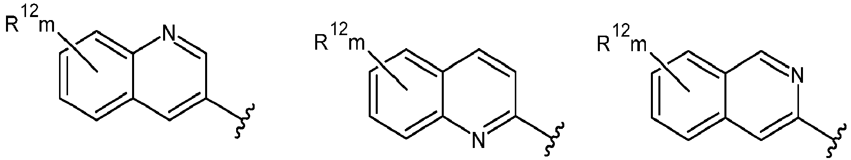 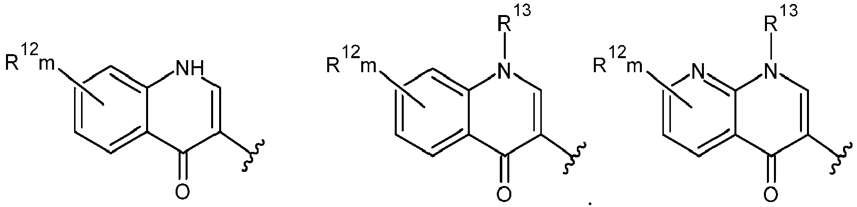 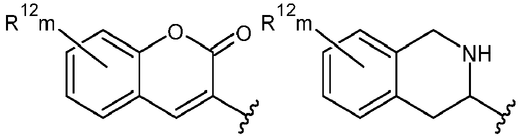
Wherein m of R¹² is any of 0 - 6, preferably 0, 1, 2, 3, 4, more preferably 0, 1, 2, 3;
Wherein R¹² is selected from -OH, -OC₁-C₆ alkyl, -C₁-C₆ alkyl, - F, -NR2, - (CH₂)ₐNR₂, -O(CH₂)ₙNR₂ with R being H or -C1-C6 alkyl and n being 1 or 2, in particular from OH, -CH₃, -C₂H₅, -OCH₃, -OC₂H₅, or two of R¹² form an acetal moiety,
Wherein R¹³ is selected from -C1-C6 alkyl.

3. Compound according to one of the preceding claims, **characterized in that** L₁ is a five membered aromatic N-heterocycle, in particular a substituted or unsubstituted imidazole or triazole, most preferably a triazole.

4. Compound according to one of the preceding claims, **characterized in that** n of R¹⁰ₙ and n of R¹¹ₙ being 0, 1, 2, 3 or 4, in particular n of R¹⁰ₙ and n of R¹¹ₙ being 0, 1, 2 or 3, and with each R¹⁰ and with each R¹¹ independently from any other R¹⁰ being selected from -OH, -OCH₃, -OC₂H₅ or -OiPr, particularly with one R¹⁰ or R¹¹ being -OH and the other R¹⁰ or R¹¹ being -OCH₃, -OC₂H₅ or -OiPr respectively.

5. Compound according to one of the preceding claims, **characterized by** the general formulae (1a) 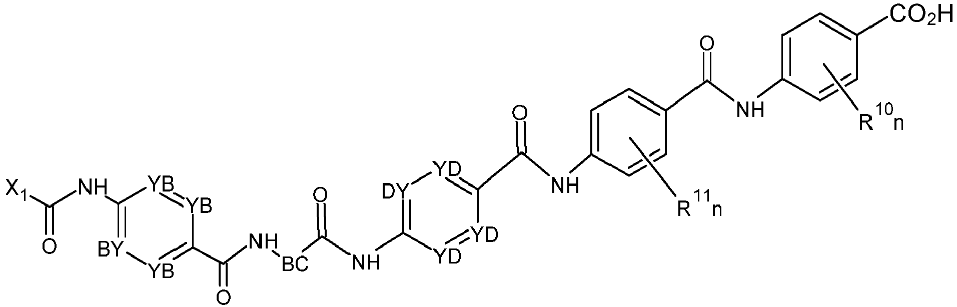
with YB being independently from each other CF, CCI or CH,
with YD being independently from each other N, CF, CCI or CH, in particular N and CH,
with X1, BC, R¹⁰n and R¹¹n as in one of the preceding claims.

6. Compound according to one of the preceding claims, **characterized by** the general formulae (2) 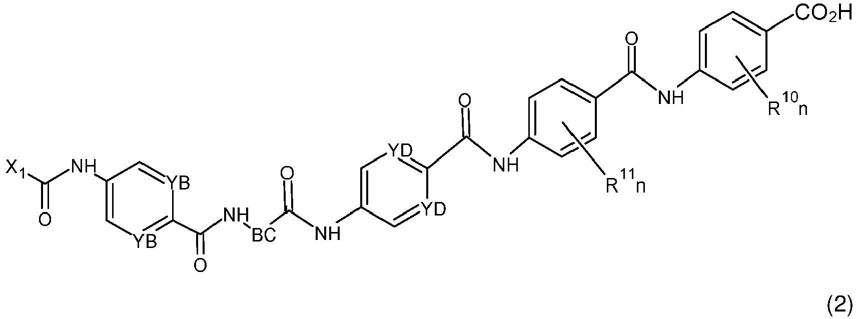
with YB being independently from each other CF, CCI or CH,
with YD being independently from each other N, CF, CCI or CH, in particular N and CH,
with X1, BC, R¹⁰n and R¹¹n as in one of the preceding claims.

7. Compound according to one of the preceding claims, **characterized by** the general formulae (3) 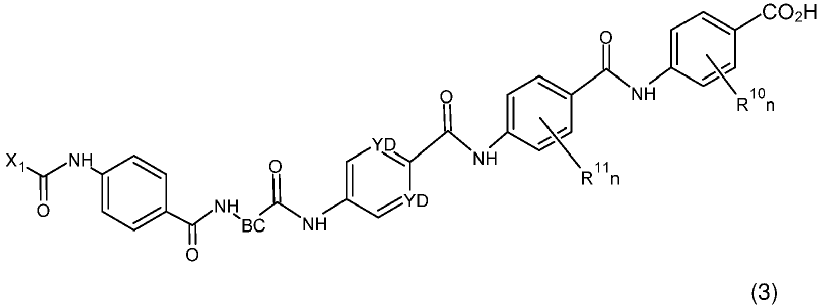 with YD being independently from each other N, CF, CCI or CH, in particular N and CH., with X1, BC, R¹⁰n and R¹¹n as in one of the preceding claims.

8. Compound according to one of the preceding claims, **characterized by** the general formulae (4) 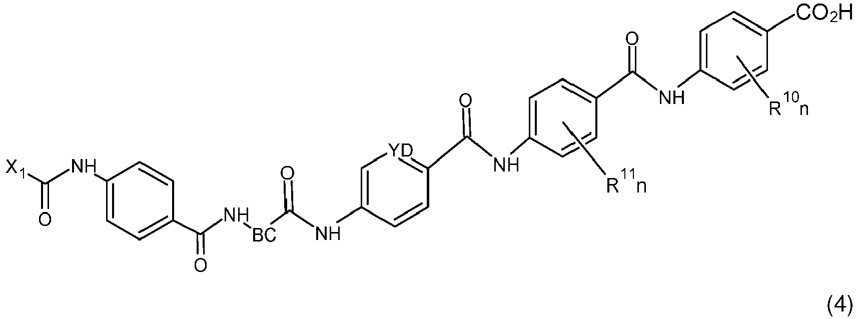
with YD being N,
with L1 being a tetrazole or imidazole, preferably tetrazole,
with X1, R¹⁰n and R¹¹n as in one of the preceding claims.

9. Compound according to one of the preceding claims, **characterized by** the general formulae (5) 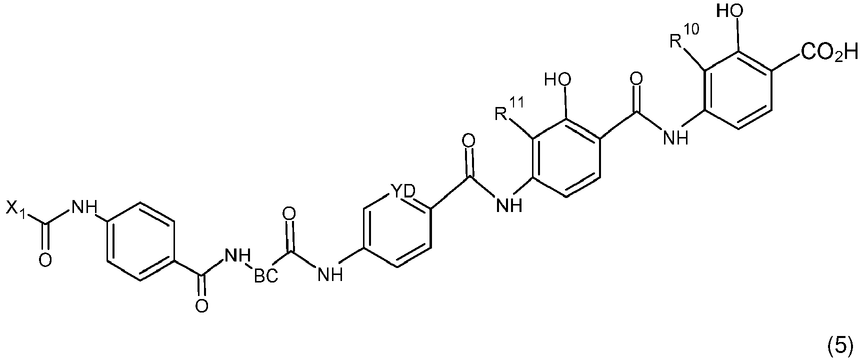
with YD being N,
with L1 being a triazolee or imidazole, preferably triazole,
with X1, R¹⁰n and R¹¹n as in one of the preceding claims.

10. Compound according to one of the claims 5-9, **characterized in that** X1 is one of the following

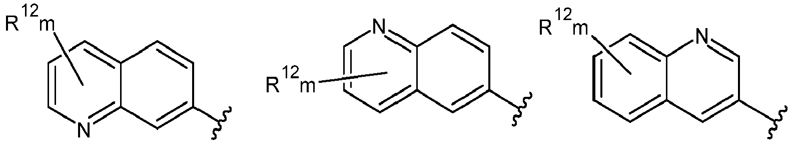

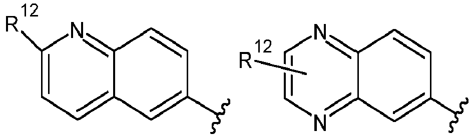

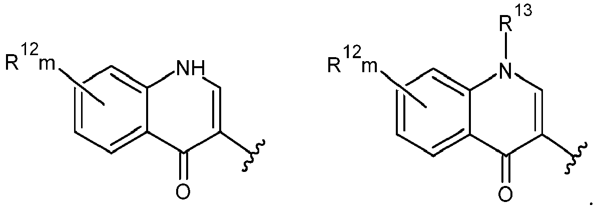

11. Compound according to one of the claims 2-10, **characterized in that**
R¹² is selected from -OH, -CH₃, -C₂H₅, -OCH₃, -OC₂H₅, in particular -OH and - CH₃,
or two of R¹² form -O-CH₂-O- moiety,

12. Compound according to one of the claims 2-11, **characterized in that** R¹³ is selected from -CH₃, -C₂H₅, -C₃H₇.

13. Compound according to one of the preceding claims, **characterized in that** X1 is one of the following

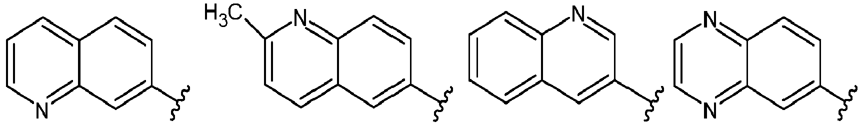

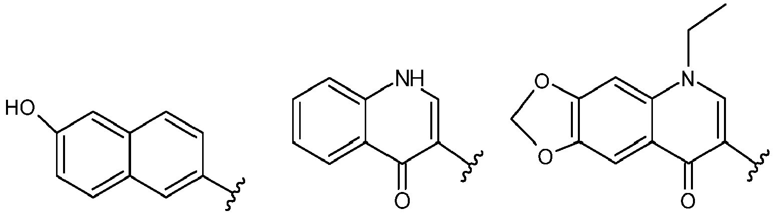

14. Compound according to one of the preceding claims for use in a method of treatment of diseases, in particular for use in a method of treatment of bacterial infections by gram-negative or gram-positive bacterial strains.

15. Compound for use in a method according to claim 14, wherein the bacterial infection is an infection by one of the genus Acinetobacter, Bordatella, Borellia, Brucella, Camphylobacter, Chlamydia, Chlamydophila, Enterobacter, Escherichia, Francisella, Haemophilus, Helicobacter, Klebisella, Legionella, Leptospira, Morganella Moraxella, Neisseria, Proteus, Pseudomonas, Rickettsia, Shigella, Salmonella, Stenotrophomonas, Treponema or Yersinia, Bacillus, Chlostridium, Corynebacterium, Enterococcus, Listeria, Micrococcus, Staphylococcus or Streptococcus Mycobacterium, Mycoplasmataceae, in particular Escherichia, Bacillus, Salmonella, Micrococcus, Mycobacterium.
